# EUROPÄISCHE PATENTSCHRIFT

(11) **EP 0 922 687 B1**
(45) Veröffentlichungstag und Bekanntmachung des Hinweises auf die Patenterteilung: **10.07.2002**
(21) Anmeldenummer: 98122544.4
(22) Anmeldetag: 01.12.1998
(51) Int. Cl.: C07C 29/141

(54) **Verfahren zur Herstellung von Alkoholen**
Process for the preparation of alcohols
Procédé pour la préparation d'alcools

(30) Priorität: 10.12.1997 DE 19754848
(43) Veröffentlichungstag der Anmeldung: 16.06.1999
(73) Patentinhaber: Celanese Chemicals Europe GmbH, 60439 Frankfurt am Main (DE)
(72) Erfinder: Frohning, Carl Dieter, Dr., 46485 Wesel (DE); Zgorzelski, Wolfgang, 46149 Oberhausen (DE); Liebern, Hans, 45478 Mülheim an der Ruhr (DE)

(56) Entgegenhaltungen:
- EP-A- 0 421 196
- EP-A- 0 537 605
- BE-A- 690 249
- DE-B- 1 643 856

## Beschreibung

Die Erfindung betrifft ein Verfahren zur Herstellung von Alkoholen durch Hydrierung von Aldehyden in der Gasphase.

Es ist bekannt, daß man Alkohole durch katalytische Hydrierung der entsprechenden gesättigten und ungesättigten Aldehyde bei erhöhter Temperatur und gegebenenfalls erhöhtem Druck herstellen kann. Die Umsetzung kann sowohl diskontinuierlich als auch kontinuierlich in homogener oder heterogener Phase durchgeführt werden. Dementsprechend wird der Hydrierkatalysator entweder in gelöster Form oder feinteilig als Suspension oder stückig als Festbettkatalysator eingesetzt. Die zu hydrierenden Verbindungen können dem Katalysator in gasförmigem oder flüssigem Zustand zugeleitet werden.

Besonders die Hydrierung von gesättigten Aldehyden, welche durch die Hydroformylierung von Alkenen erhalten werden, und die Hydrierung von α,β-ungesättigten Aldehyden, die durch die Aldolisierung von Aldehyden entstehen, besitzen hohe Bedeutung. Von großtechnischer Relevanz sind dabei insbesondere die Hydrierungen von n- und iso-Butyraldehyd, n- und iso-Valeraldehyd, Hydroxypivalaldehyd, n- und,iso-Hexanal, 2-Ethylhexenal, Gemischen isomerer Nonenale und/oder isomerer Nonanale sowie Gemischen isomerer Decenale und/oder isomerer Decanale.

Eine umfassende Darstellung bezüglich der Herstellung von Alkoholen durch katalytische Hydrierung von Carbonylverbindungen, insbesondere von Ketonen, Aldehyden und deren Derivaten findet sich in Houben-Weyl, Methoden der organischen Chemie, Georg Thieme Verlag Stuttgart-New York 1984, Band VI/1b, Seiten 9 bis 111.

Bei Hydrierungen in der Flüssigphase sind Anlagendrücke von 20 bis 300 bar üblich, um eine ausreichende Hydrierung zu erreichen. Ferner muß häufig in mehreren Stufen gearbeitet werden (DE-AS-12 31 227). Da die Reaktion stark exotherm ist, ist in technischen Reaktoren die Rückführung eines beträchtlichen Teils des hydrierten Produktes oder die Verdünnung mit einem Lösemittel zur kapazitiven Wärmeabfuhr erforderlich. Dies läßt nur verhältnismäßig niedrige Raum-Zeit-Belastungen der Reaktoren mit den Aldehyden zu, wodurch aufgrund der dann hohen Verweilzeit die Bildung von unerwünschten Folgeprodukten der reaktiven Aldehyde begünstigt wird. Diese Schwierigkeiten können durch die Hydrierung in der Gasphase vermieden werden.

Die Hydrierung von gut verdampfbaren Aldehyden wird daher vorzugsweise in der Gasphase bei erhöhten Drücken und Temperaturen in Gegenwart verschiedener, überwiegend Nickel und/oder Kupfer enthaltender Katalysatoren durchgeführt.

So ist aus EP-A-0 421 196 ein Verfahren zur Herstellung von Alkoholen bekannt, bei dem organische Carbonylverbindungen in der Gasphase mit Wasserstoff bei einer Temperatur von 60 bis 150°C und bei gegebenenfalls erhöhtem Druck in Gegenwart eines Nickel, Aluminiumoxid und Zirkondioxid enthaltenden Trägerkatalysators umgesetzt werden.

Bei der Hydrierung von Aldehyden in der Gasphase an derartigen Nickel.und/oder Kupfer enthaltenden Katalysatoren entstehen jedoch ebenfalls Nebenprodukte, die die Ausbeute an den gewünschten Alkoholen vermindern, wenn auch in geringerem Ausmaß als bei der Arbeitsweise in flüssiger Phase.

Zahlreiche Bestrebungen sind daher in diesem Gebiet darauf gerichtet, durch Weiterentwicklung der einzusetzenden Katalysatoren die Selektivität der Hydrierungsreaktion und damit die Ausbeute an Wertprodukten zu verbessern.

So ist aus der belgischen Patentschrift 690 249 ein Verfahren zur Herstellung gesättigter aliphatischer Alkohole durch katalytische Hydrierung von Aldehyden in der Gasphase bekannt, bei dem in der 1.Stufe ein Kupfer/Nickel-Katalysator auf einem Kieselgel-Träger und in der 2. Stufe ein Nickel- und/oder Palladium-haltiger Katalysator verwendet wird. Dieses Verfahren gestattet die Herstellung gesättigter Alkohole in vernünftigen Ausbeuten unter milden Bedingungen. Nachteilig ist jedoch die große Empfindlichkeit der Kieselgel-Trägerkatalysatoren, z.B. gegenüber unvorhergesehenen Störungen wie Temperaturerhöhungen oder gegenüber Verunreinigungen, die leicht zu einer dauernden Schädigung des Katalysators führen können. Insbesondere eignen sich diese Katalysatoren nicht für eine Regenerierung durch Abbrennen der Verunreinigungen bei hohen Temperaturen, da sich die Bildung von Nebenprodukten, wie Kohlenwasserstoffen und Ethern, beim Wiedereinsatz der durch eine solche Hochtemperatur-behandlung regenerierten Katalysatoren in einer Hydrierung im allgemeinen beträchtlich verstärkt.

Die Bedeutung des pH-Wertes der Oberfläche der Hydrierkatalysatoren für die Bildung unerwünschter Nebenprodukte wurde bereits früh erkannt. So ist aus Journal of Catalysis 128, 337-351 (1991) die Bildung von Ether-Nebenprodukten bei der Aldehyd-Hydrierung in Gegenwart saurer Zentren an der Oberfläche von Ni/SiO₂-Katalysatoren beschrieben. Zur Verminderung der Etherbildung wird in der DE-C-16 43 856 die Hydrierung an Kupfer und/oder Nickel enthaltenden Kieselsäuregel-Trägerkatalysatoren beschrieben, bei denen der pH-Wert der Kieselsäuregeloberfläche auf 6-10 eingestellt wird. Im Falle einer hohen Katalysatorbelastung kommt es jedoch auch bei diesen Katalysatoren in zunehmendem Maß zur Bildung gesättigter und ungesättigter Kohlenwasserstoffe, wodurch die Selektivität der Hydrierung und auch die Ausbeute an Wertprodukt abnimmt. Die ungesättigten Kohlenwasserstoffe entstehen durch Decarbonylierung, d.h. durch Abspaltung der Carbonylgruppe aus den eingesetzten Aldehyden, und besitzen somit ein Kohlenstoffatom weniger als der eingesetzte Aldehyd. Eine nachfolgende Hydrierung führt dann zur Bildung der gesättigten Kohlenwasserstoffe und von Methan aus Kohlenmonoxid. Die Hydrierung von Kohlenmonoxid zu Methan ist stark extotherm, was zu einer erhöhten Temperatur im Katalysatorbett und dadurch wiederum zur verstärkten Bildung unerwünschter Nebenprodukte führt.

Aus EP-A-0 470 344 ist eine zweistufige Hydrierung von Aldehyden bekannt, wobei in der 1. Stufe ein spezieller, alkalisch eingestellter Kupferkatalysator und in der 2.Stufe ein spezieller Nickelkatalysator eingesetzt und über 85% der Hydrierreaktion in der 1.Stufe durchgeführt werden. Beobachtet wird auch hier die Bildung von Kohlenwasserstoffen mit einem C-Atom weniger als der gewünschte Alkohol sowie von Ethern und Estern mit der doppelten C-Atom-Zahl des eingesetzten Aldehyds. Die Ester werden dabei durch eine Tischtschenko-Reaktion aus dem eingesetzten Aldehyd gebildet.

In der US-A-4,626,604 wird zur Vermeidung der Bildung der erwähnten Nebenprodukte ein mindestens dreistufiges Verfahren mit unterschiedlichen Katalysatoren zur Hydrierung ungesättigter Verbindungen beschrieben. Nachteilig an diesem Verfahren ist die außergewöhnliche Komplexität, die durch Anwendung unterschiedlicher Bedingungen für die jeweiligen Katalysatoren und deren unterschiedliche Betriebszeit verursacht wird.

Auf die Hydrierung halogenaromatischer Carbonylverbindungen in Gegenwart von Aminen an Kupferkatalysatoren geht EP-A-0 537 605 ein. Das bekannte Verfahren empfiehlt die Reaktion in einem inerten Lösungsmittel und schreibt vor, den Amingehalt nicht unter 1 Gew.-%, bezogen auf die halogenaromatische Carbonylverbindung, zu wählen. Empfohlen wird ein Amingehalt von 1,5 bis 5 Gew.-%, bezogen auf die halogenaromatische Carbonylverbindung.

Die Bildung von Kohlenwasserstoffen, Ethern, Estern und Acetalen als Nebenprodukten der Hydrierungsreaktion wirkt sich nicht nur ausbeutemindernd aus, sondern verursacht auch nicht unbeträchtliche Kosten bei der Gewinnung der reinen Alkohole, wobei insbesondere die Abtrennung der Ether wegen ihrer Siedelage besondere Schwierigkeiten bereitet und nur mit hohem Aufwand erreicht werden kann.

Zur Vermeidung der Bildung der erwähnten Nebenprodukte wurden bisher nicht nur verbesserte Katalysatoren zur Verfügung gestellt und mehrstufige Reaktionsführungen vorgeschlagen, sondern auch eine Reihe weiterer Maßnahmen entwickelt. So wirkt sich beispielsweise eine Verdünnung des in die Hydrierung eintretenden Dampfstromes, der neben den zu hydrierenden Aldehyden Wasserstoff im Überschuß enthält, vorteilhaft aus. Auf diese Weise ist es möglich, durch einen hohen Wasserstoffüberschuß bzw. eine geringe Konzentration der Aldehyde im Dampfstrom die Nebenproduktbildung zu verringern. Nachteilig bei dieser Maßnahme ist der geringe spezifische Durchsatz des zu hydrierenden Aldehyds bzw. der erforderliche hohe Wasserstoffüberschuß, der aus wirtschaftlichen Gründen im Kreis geführt werden muß. Weiterhin ist bekannt, daß der Zusatz von Wasser zu einer Verringerung der Nebenproduktbildung führen kann. Hierbei wird so verfahren, daß dem in die Hydrierung eintretenden Strom Wasserdampf in einer Konzentration von einigen Vol.-% zugesetzt wird. Dieses Wasser muß jedoch nach der Kondensation der Produkt-Alkohole wieder vollständig entfernt werden, wodurch das Verfahren aufwendig wird.

Aufgabe der Erfindung ist es daher, ein Verfahren zur Verfügung zu stellen, mit dem die Bildung von Nebenprodukten bei der Hydrierung von Aldehyden an Hydrierkatalysatoren in der Gasphase weitgehend zurückgedrängt und somit die Gewinnung der gewünschten Alkohole in hoher Selektivität und entsprechend hoher Ausbeute einfach und kostengünstig ermöglicht wird.

Gelöst wird diese Aufgabe durch ein Verfahren zur Herstellung von Alkoholen durch Hydrierung von Aldehyden in Gegenwart eines Hydrierkatalysators in der Gasphase, dadurch gekennzeichnet, daß dem zu hydrierenden Aldehyd stickstoffhaltige Basen in Mengen zwischen 1 bis 50 ppm, bevorzugt 1-25 ppm, gerechnet in ppm Stickstoff, bezogen auf den eingesetzten Aldehyd, dampfförmig dem Aldehyd zugesetzt werden.

Bei den stickstoffhaltigen Basen handelt es sich üblicherweise um primäre, sekundäre oder tertiäre Amine der Formeln I oder Diamine der Formel II

NR₃ I

R₂N-(CH₂)ₓ-NR₂ II

wobei die Reste R gleich oder verschieden und Wasserstoff, verzweigte oder unverzweigte C₂-C₁₀-Alkyl-, verzweigte oder unverzweigte C₅-C₁₀-Cycloalkylreste, verzweigte oder unverzweigte C₂-C₁₀ - Hydroxyalkylreste darstellen können und x eine ganze Zahl von 2-6 ist. Unter der Bedeutung der verzweigten oder unverzweigten C₂-C₁₀-Alkylreste steht R bevorzugt für einen Ethyl-, Propyl-, n- oder i-Butyl, n- oder i-Pentyl, Hexyl-, Heptyl- oder Octylrest. Als verzweigte oder unverzweigte C₂-C₁₀-Hydroxyalkylreste werden 2-Hydroxyethyl-, 2-Hydroxypropyl- oder 3-Hydroxypropylreste bevorzugt.

Als Diamine der obigen Formel II werden insbesondere Ethylendiamin, Propylendiamin oder 1,4-Diaminobutan verwendet, bei denen somit x gleich 2, 3 oder 4 ist und alle Reste R gleich Wasserstoff sind.

Im erfindungsgemäßen Verfahren können jedoch prinzipiell auch andere stickstoffhaltige Basen eingesetzt werden, sofern sie einen ausreichend hohen Dampfdruck besitzen, um bei den jeweils gewählten Hydrierbedingungen in Mengen zwischen 1 bis 50 ppm, bevorzugt 1-25 ppm, gerechnet in ppm Stickstoff bezogen auf den eingesetzten Aldehyd, dampfförmig dem Aldehyd zugesetzt zu werden. Bei der Hydrierung von 2-Ethylhexenal zu 2-Ethylhexanol hat sich beispielsweise der Zusatz von Tri-i-octylamin in einer Menge von 1 bis 20 ppm Stickstoff, bezogen auf den eingesetzten Aldehyd, (entsprechend 25.2-504 ppm Tri-i-octylamin) bewährt, um die Bildung von Kohlenwasserstoffen, Ethern und Estern entscheidend zu verringern.

Als Aldehyde können gesättigte oder ungesättigte Aldehyde mit 2- 10 Kohlenstoffatomen oder Gemische von diesen eingesetzt werden. Dabei können die Aldehyde in relativ reiner Form verwendet werden, oder aber auch als rohe Reaktionsprodukte, wie sie bei der Herstellung über eine Hydroformylierung, Aldolkondensation, Substitution oder Addition anfallen - gegebenenfalls auch in verdünnten Lösungen.

Beispiele für gesättigte Aldehyde sind Acetaldehyd, Propanal, n- und i- Butyraldehyd, n- und i-Pentanal, n- und i-Hexanal, n- und i-Heptanal, n- und i-Octanal, insbesondere 2-Ethylhexanal, n- und i-Nonanal, n- und i-Decanal.

Als ungesättigte Aldehyde können beispielsweise Acrolein, Crotonaldehyd, n- und i-Pentenal, n- und i-Hexenal, Hexadienal, n- und i-Heptenal, n- und i-Octenal, insbesondere 2-Ethylhexenal, n- und i-Nonenal sowie n- und i-Decenal eingesetzt werden.

Einsetzbar sind aber auch andere Aldehydderivate, die sich durch eine Reihe üblicher Synthesen, wie Aldolisierung, Aldolkondensation, Substitutions- oder Additionsreaktionen - erwähnt sei die Anlagerung von Wasser an ungesättigte Aldehyde - herstellen und mit gutem Erfolg erfindungsgemäß in die entsprechenden Alkohole überführen lassen. Bei diesen Aldehydderivaten kann es sich beispielsweise um höhermolekulare Aldehyde, Ringe enthaltende Aldehyde, bifunktionelle Aldehyde oder solche Aldehyde, die weitere funktionelle Gruppen wie z.B. Hydroxylgruppen enthalten, handeln.

Insbesondere findet das erfindungsgemäße Verfahren Anwendung auf die Hydrierung von n- und i-Butyraldehyd, n- und i- Valeraldehyd sowie 2-Ethylhexenal.

Die Hydrierung der Aldehyde kann in Gegenwart üblicher Hydrierkatalysatoren durchgeführt werden. Nickel und/oder Kupfer enthaltende Katalysatoren sowie Edelmetallkatalysatoren auf der Basis von Platin, Palladium, Rhodium oder Ruthenium haben sich besonders bewährt. Für die vollständige Hydrierung ungesättigter Aldheyde wie z.B. 2-Ethylhexenal können die aus GB 1,276,618 bekannten Nickelund/oder Palladium haltigen Katalysatoren eingesetzt werden. Die Katalysatoren können auf Trägermaterialien wie SiO₂ und/oder Al₂O₃ verschiedenster Art aufgebracht sein. Auch die aus US-A-2,549,416 bekannten, auf Zinkoxid aufgebrachten Kupferkatalysatoren können für die Gasphasehydrierung von Aldehyden verwendet werden.

Ferner können auch die zur Hydrierung von.schwefelhaltigen Ausgangsmaterialien aus Naphtha-Crackern bekannten Katalysatoren im erfindungsgemäßen Verfahren Anwendung finden. Derartige geeignete Katalysatoren sind beispielsweise aus US-A-2,709,714, US-A-2,760,994, SU 179,757 und SU 638,585 bekannt. Als Aktivatoren und Promotoren können die eingesetzten Katalysatoren ferner Oxide verschiedener 1- bis 5-wertiger Metalle enthalten. Hierbei handelt es sich z.B. um die Oxide von Zn, Mg, Mn, Cr, Zr, Fe oder von seltenen Erdmetallen. Auch Phosphate, Wolframate, Chromate, Dichromate, Molybdate, Pyro- und Polysäuren von Schwefel, Phosphor, Bor, Molybdän, Titan, Wolfram sowie deren Salze können zugegen sein. Ferner ist die Zugabe von Silber, Palladium oder Ruthenium zu Kupfer und/oder Nickel enthaltenden Katalysatoren möglich.

Weitere für das erfindungsgemäße Verfahren geeignete Katalysatoren sind beispielsweise in Hydrocarbon Processing 1993, 67 beschrieben.

Erfolgreich einsetzbar ist speziell der in der EP-A-0 421 196 beschriebene Katalysator, welcher 20-90 Gew.% Nickel, bezogen auf die Katalysatorzusammensetzung, sowie 1-30, bevorzugt 3-15 und insbesondere 4-10 Gew.-Teile Aluminiumoxid und 0.5-20, bevorzugt 1-10 und insbesondere 1.5-5 Gew.-Teile Zirkondioxid, jeweils bezogen auf 100 Gew.-Teile Nickel, als Copräzipitat auf einem Trägermaterial enthält. Als Trägermaterial eignen sich Aktivkohle, Tonerden, Bimsstein, γ-Al₂O₃, SiO₂, Kieselgel, Kieselgur und Kieselerden. Besonders haben sich SiO₂, Kieselgel, Kieselgur und Kieselerde bewährt. Üblicherweise setzt man 6-80, bevorzugt 15-65 und insbesondere 35-50 Gew.-Teile Trägermaterial je 100 Gew.-Teile Nickel ein. Die Herstellung dieser Katalysatoren ist in der EP-A- 0 421 196 beschrieben, auf die hiermit ausdrücklich Bezug. genommen wird.

Geeignet sind ferner die in der EP-A-0 604 792 beanspruchten Kupferoxid/Zinkoxid/Aluminiumoxid-Katalysatoren, die je 100 Gew.teile Kupferoxid 40-130 Gew.teile Zinkoxid, 2-50 Gew.teile Aluminiumoxid und 1-4 Gew.teile Natriumoxid enthalten, eine BET-Gesamtoberfläche von 50-100 m²/g besitzen und bei denen 75-95% der Gesamtoberfläche von Poren mit Radien von 9-1000 nm und 5-25% der Gesamtoberfläche von Poren mit Radien kleiner 9 nm gebildet werden. Auf die Beschreibung dieser Katalysatoren in der EP-A-0 604 792 wird hiermit ausdrücklich Bezug genommen.

Im erfindungsgemäßen Verfahren können auch die in der EP-A-0 618 006 beanspruchten Katalysatoren eingesetzt werden. Hierbei handelt es sich um Hydrierkatalysatoren, die aus 25-50 Gew% metallischem Nickel, 10-35 Gew% Nickeloxid, 4-12 Gew% Magnesiumoxid, 1-5 Gew% Natriumoxid und dem Rest Trägermaterial bestehen, wobei die Summe aus Nickel und Nickeloxid 40-70 Gew%, die BET-Gesamtoberfläche 80-200 m²/g und das durch Quecksilberporosimetrie bestimmte Gesamtporenvolumen 0,35-0,6 ml/g betragen, wobei 30-60 % des Gesamtporenvolumens von Poren mit einem Radius von ≤ 40 Å, 4-10 % des Gesamtporenvolumens von Poren mit einem Radius von > 40 bis 300 Å und 30-60 % des Gesamtporenvolumens von Poren mit einem Radius von > 300 bis 5000 Å gebildet werden. Auf die Beschreibung dieser Katalysatoren in der EP-A-0 618 006 wird hiermit ausdrücklich Bezug genommen.

Geeignet ist ferner der in der EP-A-0 528 305 beschriebene Hydrierkatalysator, der je 100 Gew.teile Kupferoxid 40-130 Gew.teile Zinkoxid, 2-50 Gew.teile Aluminiumoxid und gegebenenfalls 0,5-8 Gew.teile Mangan-, Molybdän-, Vanadium-, Zirkon- und/oder Erdalkalimetall-Oxid enthält und eine BET-Gesamtoberfläche von 80-175 m²/g Katalysator im nichtreduzierten Zustand besitzt, wobei 75-95% der BET-Gesamtoberfläche von Poren eines Radius rₚ ≤ 15 nm gebildet werden. Auf die Beschreibung dieser Katalysatoren in der EP-A-0 528 305 wird hiermit ausdrücklich Bezug genommen.

Zur Durchführung der Hydrierung werden der Aldehyd und die stickstoffhaltige Base gemeinsam verdampft und dann im Gemisch mit Wasserstoff über den stückigen, in einem Reaktionsgefäß fest angeordneten Katalysator geleitet. Hierbei verwendet man mindestens 2, bevorzugt 2-100 und insbesondere 3-30 mol Wasserstoff je Äquivalent des zu hydrierenden Aldehyds. Nicht umgesetzter Wasserstoff kann der Reaktion wieder zugeführt werden.

Die das Reaktionsgefäß verlassenden Dämpfe werden kondensiert und das Kondensat, falls erforderlich, durch Destillation -gegebenenfalls unter vermindertem Druckaufgearbeitet. Die Hydriertemperatur liegt im allgemeinen bei 50-250 °C, bevorzugt bei 80-160°C. Die Wahl der Hydriertemperatur wird durch die Siedelage des Aldehyds, den Druck sowie die eingesetzte Wasserstoff-Menge beeinflußt. Der Druck liegt bei 0,01-2.5 MPa und kann innerhalb dieses Bereichs unter Berücksichtigung der Siedelage sowie der eingesetzten Wasserstoff-Menge beliebig gewählt werden, unter der Voraussetzung, daß die zu hydrierenden Ausgangsstoffe und die entsprechenden hydrierten Produkte noch gasförmig vorliegen. Das erfindungsgemäße Verfahren kann kontinuierlich oder diskontinuierlich durchgeführt werden.

Bei kontinuierlicher Durchführung des Verfahrens beträgt die Raumgeschwindigkeit, ausgedrückt als Volumen flüssiges Edukt/Volumen Katalysator x Stunde (V/Vh) 0.2 bis 1.5, insbesondere 0.3 bis 1.2 und insbesondere 0.5 bis 1.0.

Überraschenderweise sind bereits sehr niedrige Konzentrationen der stickstoffhaltigen Basen von wenigen ppm, gerechnet als ppm Stickstoff bezogen auf den eingesetzten Aldehyd, ausreichend wirksam, um die Bildung der verschiedenen Nebenprodukte in der Hydrierreaktion substantiell zu verringern. Von erheblichem Vorteil ist es ferner, daß die Anwesenheit der stickstoffhaltigen Basen in niedriger Konzentration in den zur Hydrierung eingesetzten Aldehyden nicht zu den bekannten Nebenreaktionen, wie der Cannizzaro- oder Claisen-Tischtschenko-Reaktion, führt. Somit wird insgesamt die Selektivität der Hydrierung von Aldehyden erhöht.

### Beispiele :

Für alle Beispiele gilt die nachfolgende allgemeine Versuchsbeschreibung:

In einem elektrisch beheizten Mantel-Reaktor (Länge: 1500 mm, innerer Durchmesser: 20 mm) werden 150 ml (140 g) eines handelsüblichen Nickel-Katalysators (60 Gew.-% Ni, 27 Gew.-% Kieselgur, 3 Gew.-% Al₂O₃, 2 Gew.-% ZrO₂) eingefüllt.

Nach dem Aktivieren des Katalysators werden bei einer Reaktor-Manteltemperatur von 105°C und einem Druck von 0.35 MPa (abs.) stündlich 90 g n-Butanal (Reinheit 98.7%) eingepumpt. In einem dem Reaktor vorgeschalteten Verdampfer wird das n-Butanal verdampft und dampfförmig über den Katalysator geleitet. Zusammen mit dem n-Butanal wird soviel Wasserstoff (99 Vol.-% H₂; 1 Vol.-% N₂) in den Verdampfer eingespeist, daß sich eine Abgasmenge von 200 Nl/h einstellt. Die Reaktionsprodukte werden unter Reaktionsdruck auf 18°C gekühlt und in einem Abscheider in einen flüssigen und einen gasförmigen Produktstrom getrennt. Beide Produktströme werden mengenmäßig erfaßt und gaschromatographisch analysiert. Für die Berechnung der Produktverluste durch Spaltung wird davon ausgegangen, daß 1 Mol Methan pro Mol gespaltenem n-Butanal entsteht.

### Vergleichsbeispiel 1

Zum Abbau der Anfangsaktivität des Katalysators wird die Reaktion unter konstanten Bedingungen über einen Zeitraum von 180 Stunden betrieben. Nach dieser Zeitspanne werden folgende Daten ermittelt (Flüssigprodukt, Gew.-%):

| | |
|---|---|
| n-Butanal | 0,04 |
| n-Butanol | 84,91 |
| Di-n-butylether | 14,61 |
| n-Butyl-n-butyrat | 0,14 |
| Kohlenwasserstoffe | 0,30 |
| Verluste (Spaltung, Nebenprodukte): 15,2 Gew.-%, bezogen auf eingesetztes n-Butanal) | |

### Vergleichsbeispiel 2

Zur Verringerung der Nebenprodukt-Bildung werden zusätzlich zum n-Butanal (90 g/h) stündlich 9 g Wasser (10 Gew.-%, bezogen auf n-Butanal) in den Verdampfer eingespeist und gemeinsam mit verdampftem n-Butanal und Wasserstoff über den Katalysator geleitet. Nach einem Zeitraum von 266 Stunden bei dieser Arbeitsweise werden folgende Daten ermittelt (Flüssigprodukt, Gew.-%):

| | |
|---|---|
| n-Butanal | 0,10 |
| n-Butanol | 97,31 |
| Di-n-butylether | 2,31 |
| n-Butyl-n-butyrat | 0,14 |
| Kohlenwasserstoffe | 0,15 |
| Verluste (Spaltung, Nebenprodukte): 2,85 Gew.-%, bezogen auf eingesetztes n-Butanal) | |

Der Zusatz von Wasser bewirkt eine Minderung der Verluste, jedoch ist die Selektivität der Hydrierung weiterhin unbefriedigend.

### Beispiel 1

Es werden 90 g/h n-Butanal in den Reaktor eingespeist. Dem n-Butanal wird Tri-iso-octylamin in einer Menge von 250 ppm (0,025 Gew.-%, bezogen auf n-Butanal entsprechend 9.9 ppm Stickstoff bezogen auf eingesetztes n-Butanal) zugesetzt. Nach einer Betriebszeit von 158 Stunden werden folgende Daten ermittelt (Flüssigprodukt, Gew.-%):

| | |
|---|---|
| n-Butanal | 0,13 |
| n-Butanol | 99,31 |
| Di-n-butylether | 0,011 |
| n-Butyl-n-butyrat | 0,02 |
| Kohlenwasserstoffe | 0,53 |
| Verluste (Spaltung, Nebenprodukte): 0,61 Gew.-%, bezogen auf eingesetztes n-Butanal) | |

## Patentansprüche

1. Verfahren zur Herstellung von Alkoholen durch Hydrierung von Aldehyden in Gegenwart eines Hydrierkatalysators in der Gasphase, **dadurch gekennzeichnet, daß** dem zu hydrierenden Aldehyd stickstoffhaltige Basen in Mengen zwischen 1 bis 50 ppm, bevorzugt 1-25 ppm, gerechnet in ppm Stickstoff, bezogen auf den eingesetzten Aldehyd, dampfförmig dem Aldehyd zugesetzt werden.

2. Verfahren nach Anspruch 1, **dadurch gekennzeichnet, daß** es sich bei den stickstoffhaltigen Basen um primäre, sekundäre oder tertiäre Amine der Formel I oder Diamine der Formel II
NR₃ I
R₂N-(CH₂)ₓ-NR₂ II
handelt, wobei die Reste R gleich oder verschieden und Wasserstoff, verzweigte oder unverzweigte C₂-C₁₀-Alkyl-, verzweigte oder unverzweigte C₅-C₁₀-Cycloalkylreste, verzweigte oder unverzweigte C₂-C₁₀-Hydroxyalkylreste darstellen können und x eine ganze Zahl von 2 - 6 ist.

3. Verfahren nach Anspruch 2, **dadurch gekennzeichnet, daß** R unter der Bedeutung der verzweigten oder unverzweigten C₂-C₁₀-Alkylreste für einen Ethyl-, Propyl-, n- oder i-Butyl, n- oder i-Pentyl, Hexyl-, Heptyloder Octylrest steht, unter der Bedeutung der verzweigten oder unverzweigten C₂-C₁₀ - Hydroxyalkylreste einen 2-Hydroxy-ethyl-, 2-Hydroxypropyl- oder 3-Hydroxypropylrest darstellt und in Diaminen der Formel II x gleich 2, 3 oder 4 ist und alle Reste R gleich Wasserstoff sind.

4. Verfahren nach einem oder mehreren der Ansprüche 1-3, **dadurch gekennzeichnet, daß** als Aldehyde gesättigte oder ungesättigte Aldehyde mit 2 - 10 Kohlenstoffatomen oder Gemische davon eingesetzt werden.

5. Verfahren nach Anspruch 4, **dadurch gekennzeichnet, daß** als gesättigte Aldehyde Acetaldehyd, Propanal, n-und i- Butyraldehyd, n- und i-Pentanal, n- und i-Hexanal, n- und i-Heptanal, n- und i-Octanal, insbesondere 2-Ethylhexanal, n- und i-Nonanal oder n- und i-Decanal, als ungesättigte Aldehyde Acrolein, Crotonaldehyd, n- und i-Pentenal, n- und i-Hexenal, Hexadienal, n- und i-Heptenal, n- und i-Octenal, insbesondere 2-Ethylhexenal, n- und i-Nonenal oder n-und i-Decenal eingesetzt werden.

6. Verfahren nach einem oder mehreren der Ansprüche 1-5, **dadurch gekennzeichnet, daß** die Hydrierung der Aldehyde in Gegenwart eines Nickel und/oder Kupfer enthaltenden Katalysators oder eines Edelmetallkatalysators auf der Basis von Platin, Palladium, Rhodium oder Ruthenium durchgeführt wird, welcher auf Trägermaterialien aufgebracht ist.

7. Verfahren nach Anspruch 6, **dadurch gekennzeichnet, daß** der Katalysator 20-90 Gew.% Nickel, bezogen auf die Katalysatorzusammensetzung, sowie 1-30, bevorzugt 3-15 und insbesondere 4-10 Gew.-Teile Aluminiumoxid und 0.5-20, bevorzugt 1-10 und insbesondere 1.5-5 Gew.-Teile Zirkondioxid, jeweils bezogen auf 100 Gew.-Teile Nickel, als Copräzipitat auf einem Trägermaterial enthält.

8. Verfahren nach einem oder mehreren der Ansprüche 1-7, **dadurch gekennzeichnet, daß** der Aldehyd und die stickstoffhaltige Base gemeinsam verdampft und dann im Gemisch mit Wasserstoff über den stückigen, in einem Reaktionsgefäß fest angeordneten Katalysator geleitet werden, wobei mindestens 2, vorzugsweise 2-100 und insbesondere 3-30 mol Wasserstoff je Äquivalent des zu hydrierenden Aldehyds eingesetzt werden, die Hydriertemperatur bei 50-250°C, bevorzugt bei 80-160°C und der Druck bei 0,01-2.5 MPa liegt.

9. Verfahren nach einem oder mehreren der Ansprüche 1-8, **dadurch gekennzeichnet, daß** es kontinuierlich durchgeführt wird und die Raumgeschwindigkeit, ausgedrückt als Volumen flüssiges Edukt/Volumen Katalysator x Stunde (V/Vh), 0.2 bis 1.5, insbesondere 0.3 bis 1.2 und insbesondere 0.5 bis 1.0 beträgt.

## Claims

1. A process for preparing alcohols by hydrogenation of aldehydes in the presence of a hydrogenation catalyst in the gas phase, wherein nitrogen-containing bases in amounts of from 1 to 50 ppm, preferably 1-25 ppm, calculated in ppm of nitrogen based on the aldehyde used, are added in vapor form to the aldehyde to be hydrogenated.

2. Process as claimed in claim 1, wherein the nitrogen-containing bases are primary, secondary or tertiary amines of the formula I or diamines of the formula II
NR₃ I
R₂N-(CH₂)ₓ-NR₂ II
where the radicals R can be identical or different and can be hydrogen, branched or unbranched C₂-C₁₀-alkyl radicals, branched or unbranched C₅-C₁₀-cycloalkyl radicals, branched or unbranched C₂-C₁₀-hydroxyalkyl radicals and x is an integer from 2 to 6.

3. The process as claimed in claim 2, wherein branched or unbranched C₂-C₁₀-alkyl radicals R are ethyl, propyl, n- or i-butyl, n- or i-pentyl, hexyl, heptyl or octyl radicals, branched or unbranched C₂-C₁₀-hydroxyalkyl radicals R are 2-hydroxyethyl, 2-hydroxypropyl or 3-hydroxypropyl radicals and in diamines of the formula II x is 2, 3 or 4 and all radicals R are hydrogen.

4. The process as claimed in one or more of claims 1-3, wherein the aldehydes used are saturated or unsaturated aldehydes having 2-10 carbon atoms or mixtures thereof.

5. The process as claimed in claim 4, wherein the saturated aldehydes used are acetaldehyde, propanal, n- and i-butyraldehyde, n- and i-pentanal, n- and i-hexanal, n- and i-heptanal, n- and i-octanal, in particular 2-ethylhexanal, n- and i-nonanal or n- and i-decanal and the unsaturated aldehydes used are acrolein, crotonaldehyde, n- and i-pentenal, n- and i-hexenal, hexadienal, n- and i-heptenal, n- and i-octenal, in particular 2-ethylhexenal, n- and i-nonenal or n- and i-decenal.

6. The process as claimed in one or more of claims 1-5, wherein the hydrogenation of the aldehydes is carried out in the presence of a nickel- and/or copper-containing catalyst or a noble metal catalyst based on platinum, palladium, rhodium or ruthenium, which catalyst is applied to a support material.

7. The process as claimed in claim 6, wherein the catalyst comprises 20-90% by weight of nickel, based on the catalyst composition, and also 1-30, preferably 3-15 and in particular 4-10, parts by weight of aluminum oxide and 0.5-20, preferably 1-10 and in particular 1.5-5, parts by weight of zirconium dioxide, in each case based on 100 parts by weight of nickel, as coprecipitate on a support material.

8. The process as claimed in one or more of claims 1-7, wherein the aldehyde and the nitrogen-containing base are vaporized together and then passed in admixture with hydrogen over the granular/pelletized catalyst arranged as a fixed bed in a reaction vessel, where at least 2 mol, preferably 2-100 mol and in particular 3-30 mol, of hydrogen are used per equivalent of the aldehyde to be hydrogenated, the hydrogenation temperature is 50-250°C, preferably 80-160°C, and the pressure is 0.01-2.5 MPa.

9. The process as claimed in one or more of claims 1-8 carried out continuously at a space velocity, expressed as volume of liquid starting material/volume of catalyst x hour (V/Vh), of from 0.2 to 1.5, preferably from 0.3 to 1.2 and in particular from 0.5 to 1.0.

## Revendications

1. Procédé de production d'alcools par hydrogénation d'aldéhydes en présence d'un catalyseur d'hydrogénation en phase gazeuse **caractérisé en ce que** des bases azotées sont ajoutées sous forme gazeuse à l'aldéhyde à hydrogéner en des quantités situées entre 1 et 50 ppm, de préférence 1 et 25 ppm, calculées en ppm d'azote, par rapport à l'aldéhyde utilisé.

2. Procédé selon la revendication 1, **caractérisé en ce que** les bases azotées sont des amines primaires, secondaires ou tertiaires de formule I ou des diamines de formule II
NR₃ I
R₂N-(CH₂)ₓ-NR₂ II
où les restes R sont identiques ou différents et peuvent représenter l'hydrogène, des restes alkyle en C₂-C₁₀ ramifiés ou non ramifiés, des restes cycloalkyle en C₅-C₁₀ ramifiés ou non ramifiés, des restes hydroxyalkyle en C₂-C₁₀ ramifiés ou non ramifiés, et x est un nombre entier 2 à 6.

3. Procédé selon la revendication 2, **caractérisé en ce que**, quand il représente des restes alkyle en C₂-C₁₀ ramifiés ou non ramifiés, R représente un reste éthyle, propyle, n- ou i-butyle, n- ou i-pentyle, hexyle, heptyle ou octyle, et quand il représente des restes hydroxyalkyle en C₂-C₁₀ ramifiés ou non ramifiés, R représente un reste 2-hydroxyéthyle, 2-hydroxypropyle ou 3-hydroxypropyle, et, dans les diamines de formule II, x est égal à 2, 3 ou 4 et tous les restes R sont l'hydrogène.

4. Procédé selon une ou plusieurs des revendications 1 à 3, **caractérisé en ce que** l'on utilise comme aldéhydes des aldéhydes saturés ou insaturés ayant 2 à 10 atomes de carbone ou des mélanges de ceux-ci.

5. Procédé selon la revendication 4, **caractérisé en ce que** l'on utilise comme aldéhydes saturés l'acétaldéhyde, le propanal, le n- et i-butyraldéhyde, n- et i-pentanal, le n- et i-hexanal, le n- et i-heptanal, le net i-octanal, en particulier le 2-éthylhexanal, le n- et i-nonanal ou le n- et i-décanal, comme aldéhydes insaturés l'acroléine, le crotonaldéhyde, le net i-penténal, le n- et i-hexénal, l'hexadiénal, le n- et i-hepténal, le n- et i-octénal, en particulier le 2-éthylhexénal, le n- et i-nonénal ou le n- et i-décénal.

6. Procédé selon une ou plusieurs des revendications 1 à 5, **caractérisé en ce que** l'on conduit l'hydrogénation des aldéhydes en présence d'un catalyseur contenant du nickel et/ou du cuivre ou d'un catalyseur à métal noble à base de platine, de palladium, de rhodium ou de ruthénium qui est appliqué sur des supports.

7. Procédé selon la revendication 6, **caractérisé en ce que** le catalyseur contient 20-90 % en masse de nickel, par rapport à la composition du catalyseur, ainsi que 1-30, de préférence 3-15 et en particulier 4-10 parties en masse d'oxyde d'aluminium et 0,5-20, de préférence 1-10 et en particulier 1,5-5 parties en masse de dioxyde de zirconium, dans chaque cas par rapport à 100 parties en masse de nickel, sous forme de coprécipité sur un support.

8. Procédé selon une ou plusieurs des revendications 1 à 7, **caractérisé en ce que** l'aldéhyde et la base azotée sont évaporés conjointement puis sont amenés à passer en mélange avec l'hydrogène au-dessus du catalyseur en morceaux disposé de manière fixe dans un récipient réactionnel, où au moins 2, de préférence 2-100 et en particulier 3-30 mol d'hydrogène sont utilisées par équivalent de l'aldéhyde à hydrogénér, la température d'hydrogénation est située à 50-250°C, de préférence à 80-160°C et la pression est située à 0,01-2,5 MPa.

9. Procédé selon une ou plusieurs des revendications 1 à 8, **caractérisé en ce qu'**il est mis en oeuvre en continu et la vitesse spatiale, exprimée en volume d'éduit liquide/volume de catalyseur x h (V/Vh), est de 0,2 à 1,5, en particulier de 0,3 à 1,2 et en particulier de 0,5 à 1,0.
